# EUROPEAN PATENT SPECIFICATION

(11) **EP 1 248 237 B1**
(45) Date of publication and mention of the grant of the patent: **31.05.2006**
(21) Application number: 02251816.1
(22) Date of filing: 14.03.2002
(51) Int. Cl.: G06T 7/20, G06T 17/40

(54) **Method for measuring volumetric changes in portions of a human body**
Verfahren zum Messen volumetrischer Veränderungen an menschlichen Körperteilen
Procédé de mésure des changements volumetriques des portions du corps humain

(30) Priority: 14.03.2001 US 275733 P; 20.07.2001 US 306776 P; 20.02.2002 US 77997
(43) Date of publication of application: 09.10.2002
(73) Proprietor: JOHNSON & JOHNSON CONSUMER COMPANIES, INC., Skillman, New Jersey 08558-9418 (US)
(72) Inventor: Wallo, Warren, Belle Mead, NJ 08502 (US); Kollias, Nikiforos, Skillman, NJ 08558 (US)
(74) Representative: Fisher, Adrian John

(56) References cited:
- US-A- 5 482 048
- US-A- 5 687 259
- US-A- 5 852 672
- S. JASPERS, H. HOPERMANN, G. SAUERMANN, U. HOPPE, R. LUNDERSTÄDT AND J. ENNEN: "Rapid in vivo measurement of the topography of human skin by active image triangulation using a digital micromirror device" SKIN RESEARCH AND TECHNOLOGY, vol. 5, 1999, pages 195-207, XP001119021

## Description

### BACKGROUND OF THE INVENTION

### 1. Field of the Invention

The present invention relates to a method for demonstrating and measuring changes in portions of a human body. The method utilizes three-dimensional analysis to identify and measure changes in areas of the body such as the face, arms, and legs. The method is particularly useful for demonstrating the effectiveness of cosmetic products at lifting the face, contouring the face and reducing wrinkles.

### 2. Description of the Prior Art

We are living in an era of increased awareness relating to physical health. In general, people want to be and look healthier than ever before. The role of exercise in good physical health is well understood. This has led to increased enrollment at health clubs. Many employers are even offering health club benefits at work.

The role of proper nutrition in good physical health is also well understood. There has been a significant change in eating habits directed towards better physical health. The combination of eating well and exercising generally leads to weight loss. As a result, the person tends to feel and look healthier.

As people age, they tend to show signs of aging, such as their hair turning gray, their skin sagging, they develop age spots and wrinkles on their skin and they have increased skin surface roughness. While exercise and proper nutrition promote good health, they don't eradicate the signs of aging. Therefore, many people rely on cosmetics to reduce wrinkles, improve skin pigmentation and decrease skin texture.

The manufacturers of cosmetics strive to develop products that demonstrate improvements in lifting the skin, reducing wrinkles, improving skin pigmentation and the like to meet the needs of aging people. To our knowledge, there is no accurate method for demonstrating and measuring these improvements. Therefore, there is a need for a method of demonstrating and measuring changes in portions of a human body.

Jaspers, et. al., "Rapid in vivo measurement of the topography human skin by active image triangulation using a digital micromirror device", in Skin Research and Technology, Volume 5, Number 3, pages 195-207 (August 1999), describe a method for measuring topography of skin. This method utilizes 3D imaging and is taught to possibly be useful for use in the cosmetics industry.

US-A-5 687 259 discloses an imaging system for cosmetic surgery patients. Two-dimensional images may be overlaid.

United States Patent No. 5,867,588 describes a method for analyzing facial configurations and components. In this patent, an image of the face is obtained which is then analyzed with complex mathematical algorithms to create a composite of pentagons. Using selected points and lines, an overlay system is constructed that defines the face. In addition, selected line segments are combined to form the features of an ideal human face. The features of the computer-derived representation of an ideal human face are compared with the actual features of the subject's face. The mathematical information obtained can be used as a guide in application of cosmetics, as a pre-surgical aid for planning plastic and re-constructive surgery, or as a standard for analyzing the face for academic study or for quantifying the features of the face for use in an identification system.

Plastic surgeons perform many operations directed at eradicating symptoms of aging. Face lifts, tummy tucks, chest lifts and the like are common procedures. These procedures generally result in drastic changes to portions of the human body. The improvements obtained from cosmetics are subtle in comparison to those obtained from surgery. We believe that plastic surgeons utilize three-dimensional imaging to plan surgeries and to demonstrate the results of the surgery through before and after comparisons. To our knowledge, overlaying of three-dimensional images has not been utilized to demonstrate or measure the effectiveness of cosmetics on portions of the human body or as a diagnostic tool for monitoring three-dimensional changes on the body.

### SUMMARY OF THE INVENTION

Our invention provides a method for measuring visible changes in a portion of a human body including: obtaining a first three-dimensional image of the portion of a human body; treating the portion of a human body to create changes therein or providing a time period between a first and second image is obtained; obtaining a second three-dimensional image of the portion of a human body so treated; overlaying the first three-dimensional image and the second three-dimensional image; and comparing the first and second images to measure visible changes in the portion of a human body.

In a second embodiment, the present invention provides a method of measuring three-dimensional changes in a portion of a human body, the method comprising:
obtaining a first three-dimensional image of the portion of a human body;
obtaining a subsequent three-dimensional image or images of the portion of a human body;
overlaying the first three-dimensional image and the second three-dimensional image; and
comparing the first and second images to measure three-dimensional changes in the portion of a human body,
wherein the changes are due to passage of time, stress, aging, diet, fatigue, environmental insult, disease, injury, or surgery or the like.

As used herein, the term "measure" includes, but is not limited to, any visualization, quantification, instrumental output or fabrication of surface so obtained, which represents a change in the three dimensional spaces. These changes may include, but are not limited to, volumetric changes, contour changes, firmness changes, plumping changes, aging changes, texture changes, area changes, depth changes, elevation changes, size changes, shape changes, distribution changes and tone changes. The term "treat" includes, but is not limited to, applying some type of external or internal medicament, cosmetic composition, or other activity that effects changes or is intended to effect changes in the portion of the human body of which said image is obtained.

Although three-dimensional ("3D") is referred to herein, the technique utilized herein was technically 2.5-dimensional, meaning that we have a single z value at each x and y. True 3D contains a multitude of z values at each x and y and is also useful in the method of the present invention.

### DETAILED DESCRIPTION OF PREFERRED EMBODIMENTS

The first step in the method of the invention is to obtain a first three-dimensional image of the portion of a human body. Numerous commercial devices are available to obtain three-dimensional images of objects. Any such device may be used in the process of the invention. Two commercially available devices are described herein.
The Minolta 3D 1500 camera, commercially available from the Minolta Corporation, is an easy to use, moderate resolution device. The PRIMOS non-contact optical profiling instrument is more complex, but provides much higher resolution images. However, any three-dimensional imaging device that provides such three dimensional images may be utilized.

The Minolta 3D 1500 camera may be used to acquire images of subjects. The 3D 1500 emits two flashes in a single shot and records one "image without a projected stripe" and one "image with projected stripes" for the same subject. That is, it takes an ordinary flash photo on the first flash and projects the stripes on the second flash. The two images of data are transferred to a computer where a three-dimensional image is generated from the images by a software application called MetaFlash Studio. This software is available in connection with the Minolta 3D 1500 camera, but may be purchased separately. However, any available software application capable of creating three-dimensional objects from two-dimensional images may be utilized in connection with translating images from a two-dimensional rendering into a three-dimensional rendering.

The Minolta camera should be oriented to project horizontal lines across the face. One data acquisition should be made at baseline and after product usage for each subject, with the photographer carefully watching the subject during the flashes. In cases where a blink was observed or suspected, a second photo should be taken to assure that a satisfactory image would be obtained. The data should be transferred to an ordinary PC and the file should be labeled with the date and subject number. A list of the subject order should be kept to correlate with the Minolta numbering system.
MetaFlash software may be used as described below to obtain a three-dimensional image.

### Procedure for Acquiring 3D Images from camera using MetaFlash software

Before processing and editing an image, it must first be acquired from the raw data. Certain options and settings in the model reconstruction parameters can improve the quality of the picture.

### Description of the Settings used for Acquiring the 3D images

For optimum quality images, the following settings may be utilized: Under "Resolution," the *Generate Dynamic Resolution* option was unchecked. The *Maximum Number of Triangles* was set to be 300,000. This prevents the acquisition of too few triangles in the image, which would yield insufficient resolution and inaccurate depth. In the "Output Quality" category, the *Output Pixel Step* value is preferably set to 10, which represents a ratio of 1:10. This will result in 10,000-20,000 triangles for images using grades. The value for *X Tolerance for Pixel Step* may be set at the default value of 3. In the "Normal Smooth" box, the setting of 0.2 for *Alpha* and 1 *Iteration* should be used. The "3D Texture Correction" option may not be checked. Under "Texture," both the *Clip Unused* Area and *Auto Exposure* may be checked, and "No Limit" may be chosen in the *Maximum.Texture Size.* The "Object Outline" box may then be unchecked. In the "Heap Smooth" category, the number of *Iterations* may be set at 1. The *Use Grades* option is preferably selected to approximately double the number of triangles in the image. *Motion Compensation* is preferably checked. The *Blow Average Link Length* is preferably set to 0.8. The *3D Filtering Length* is preferably usually set at the default of 15 (cm). If one desires to exclude points where the vertices are farther than a shorter distance, this value can be set to that distance (in centimeters). The *Background Removal Threshold* is preferably set at 5.

Phase-shifting Rapid In-vivo Measurement Of Skin (PRIMOS) is a new imaging technology developed by GFMesstechnik, Germany is a white light non-contacting optical profiler. PRIMOS is able to capture surface topography of an object at high resolution without contact by projecting a series of B/W fringes which are shifted over the surface of the object. Displacement of the fringes from where they would be for a flat object is determined by the camera and the amount of displacement is proportional to the elevation changes on the surface. For visualization purposes, the camera image may be wrapped on the height data to generate a dimensional photograph.

### Preparing the Images for Analysis:

(1) 3-D images of the subjects may be made using the PRIMOS system (GFMesstechnik Product #: 207.02:000.00).
   The settings that are preferably used are as follows: Hardware Video Gain: 16,
   Software Video Gain: 10, Partial Gray Code Wavelength: 16, # Pics: 6, Crosshair positioning: Even with eye level on bridge of nose
(2) The images may be preferably processed using IDL® , a software package useful for manipulating three-dimensional images commercially available from Research Systems, Inc. (Boulder, Colorado), to eliminate noise contained on the outside edges of the images, to reduce the file size of the images to allow for more reasonable computer processing time and to convert from .sdf to .obj for use in other 3D software programs.

The second step in the process of this invention is preferably treating the portion of a human body to create changes, although the process of this invention may also be applied to observe changes that occur without treatment as well, in that the body may change over time.

However, as to changes that take place in response to treatment, such changes are typically seen in the contour or firmness therein. Any treatment that results in a change to the portion of the human body may be utilized. Suitable treatments include, but are not limited to, an exercise regimen, the application of topical skin care agents, ingestion of oral skin care agents, drugs and combinations thereof. Any portion of the human body may be monitored for changes. Suitable portions of the human body include, but are not limited to, the head, face, neck, lips, cheeks, eyes, forehead, hair, chest, arms, waist, legs, knees, ankles, feet, and buttocks. This can be applied to conditions of the body such as, but not limited to, aging, growth (e.g. monitoring growth rates in children), sagging, changes in firmness, changes in contour, cellulite, scarring, changes in the size and shape of pores, changes in pimples and other skin diseases and conditions, and changes in skin lesions.

Any exercise regimen that results in a change to a portion of a human body may be useful in the method of the invention. The exercise regimen may include walking, jogging, swimming, bicycling, weight lifting, and combinations thereof.

Any topical or oral skin care agent that provides a change to a portion of a human body may be used in the process of the invention. Suitable skin care agents include, but are not limited to, inorganic sunscreens such as titanium dioxide and zinc oxide; organic sunscreens such as octyl-methyl cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acid such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucopehtonic acid, glucopheptono 1,4-lactone, gluconic acid, gluconolactone, glucoronic acid, glucurronolactone, glycolic acid, isopropyl pyruvate, methyl pyruvate, mucic acid, pyruvic acid, saccharic acid, saccharic acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, safflower, dimethylaminoethanol, derivatives thereof, analogues thereof, and mixtures thereof. Preferred anti-aging agents include retinoids, anti-oxidants, alpha-hydroxy acids, dimethylaminoethanol, and beta-hydroxy acids.

The topical skin care agent may be formulated with water, surfactants, glycols and the like by means well known in the art. Particularly useful formulations and skin care agents are described in US-B-6 762 158.

The third step in the method of the invention is to obtain a subsequent three-dimensional image or images of the portion of a human body so treated. Such a step, even with regard to two-dimensional images, in the past has proved to be challenging due to the difficulty of duplicating the precise conditions of an earlier image.
The pose, the lighting, the expression, the extent to which muscles are contracted or relaxed and three-dimensional positioning all need to be the same in both images in order to be able to compare them.

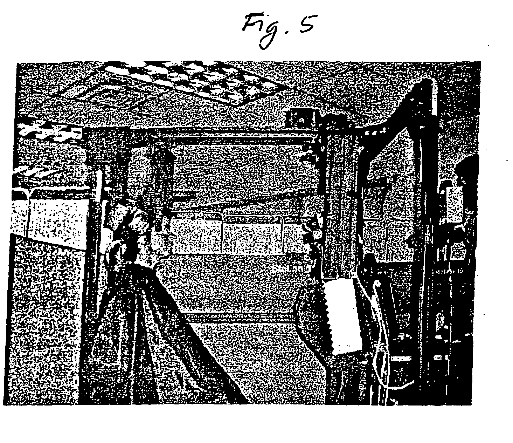

We have discovered a novel means for achieving reproducible positioning in connection with the methods of this invention. In order to obtain reproducible positioning and facial expression, images of seated volunteers were taken. Two basic setups are preferably utilized for this purpose. One setup preferably utilizes a head-restraint with the subject resting his or her chin gently in the chin-rest. A newly designed custom setup may also be preferably employed that supports the head from the back and the top to provide a full view of the face without distorting the front of the face where the skin measurements are to be made. A device such as that set forth in Figure 5 may preferably be used to maintain the position of the subject during the first and during the second imaging procedure. The subject may be placed in a chair and his or her head tilted back to rest on a headrest. A forehead brace is then placed such that it surrounds the subject's head on the top and both sides. A curtain may be drawn around the apparatus in order to provide a shadow-free environment.

However, maintaining reproducible positions may also be achieved by utilizing software that can rotate and adjust the image to create the same positioning at two separate time periods. The MetaFlash software, for example, may be utilized in this way, as set forth hereinabove.

The following procedure was developed to standardize the positioning of the face and the expression on the face: subjects were imaged individually in a quiet room with subdued lighting. Black elastic headbands may be used to hold hair and bangs away from the face during measurements. Care should be taken to ensure that the elastic headbands do not distort the skin in any way, but only lightly hold back the hair. The position of the face in the chinrest should be monitored with respect to the chin, forehead, nose and eye location, prior to imaging. The PRIMOS device projects a pattern onto the face for added positional alignment. At subsequent time points, the imaging analyst should view the baseline image and make final positional adjustments to achieve extreme consistency. The subject should be instructed to stare at a pre-positioned dot when faces are imaged with the eyes open. They should be instructed to remain still during the imaging and informed of the lighting or flashes that would occur during the data acquisition. The subject should preferably be instructed in a gentle voice to relax his or her face to remove any traces of smile or frown (or other section of the body being imaged). The analyst should wait about 5 to about 10 seconds and then image the subject.
Comparison of the new image with the baseline image should be performed to ensure consistency. Repeat measurements should be performed when movement, blinking or misalignment is observed. The second image is preferably obtained with the same procedure and the same equipment described above for taking the first image.

The next step in the method of the invention is to compare before- and after-treatment images. There are a variety of software products for analyzing and comparing three-dimensional images. Any such software can be used for the process of the invention. For optimal analysis, combinations of software and custom software may be useful.

### Using Minolta MetaFlash Imaging Technology to Overlay Faces

We discovered that MetaFlash can overlay two images and they can be rotated on any axis to analyze different aspects of the object, thus allowing a face comparison from almost any angle. This can be accomplished by zooming the two images in different windows until they are of equal size and then positioning the windows on top of each other. Then for a more precise overlay, each image can be rotated so that they are in the same orientation. One can toggle between the windows and observe any changes between the two images.

However, one problem in attaining a perfect overlay is that the program does not save the position of the image on the x-y coordinate plane. Object tools and camera tools allow panning around the image in this plane, rotating the image, and zooming into and out of the image. However, the software only allows the changes in object rotation (home position) to be saved to file.
Therefore, any changes made to the x-y shifting or zooming of the object or camera angle are not saved.
This poses a huge problem in creating an overlay if, when taking the photograph of an image, the object is not positioned the same in both frames. This would result in images in two windows that may have the same orientation, but are not laid on top of each other and cannot be viewed correctly without a manual repositioning every time the file is reopened. Although measurements of changes can be made utilizing this technique, it would be easier to be able to toggle between two overlaid images.

An alternate method of toggling between two overlaid images was determined utilizing a feature called Solo Mode. In Solo Mode, two objects that are open in the same window can be viewed one at a time by toggling between the images. However this option also uses an automatic positioning method which cannot be directly controlled. This option maximizes the boundary box (with respect to the x, y, and z-axes) of a single object in a window. The boundary box is a rectangular space enveloping the object. Its dimensions are determined by the boundaries of the actual 3-dimensional object when the photograph is taken. The properties of the 3-D object are determined by the area marked by a continuous, non-dark surface, and are bounded by any dark areas surrounding it. The problem in working with Solo Mode is that the camera is not always consistent in determining what parts of the object are non-dark, as well as the depth of the object. This results in two photos of the same object having slightly different sized boundary boxes. Though the orientation of the objects can be manipulated to be the same, Solo Mode will portray these objects as different sized or in different positions. Again, although this method can be used to measure changes, it is not an optimum method.

This problem was solved by using the slicing plane to cut unneeded extremities of the object in order to equalize the dimensions of the box. The logic behind this method is to shorten certain axes of the object's boundary box so that Solo Mode will compensate for the loss by shifting the object in a desired direction.
Cutting in the z-axis zooms the object in or out, cutting the y-axis shifts the object up or down, and cutting the x-axis shifts the object left or right. For example, if an object is positioned slightly above the second object when toggling between windows in Solo Mode, a direct slice from the bottom boundary will lower that object. A slicing plane parallel to the x-y plane that cuts behind (or in front) of the head will enlarge an object. Usually the image of a face will have some unneeded regions far into the z-axis from hair or ears.
These can be cut away to start to equalize the size. By toggling between the two images in Solo Mode, the smaller object is selected and edited first. This way, any cut that slices some of the z-axis will also increase the size to match the larger image. This procedure is followed until both objects appear to be the same size in Solo Mode and are positioned directly on top of each other.

The above procedure was used to create composite three-dimensional images for subjects in our clinical study.

These three-dimensional images were archived on CD with subject number and time points.

### Exporting images to an appropriate viewing format

The MetaFlash software provides an excellent view of the 3D image of one subject at a time. However, it is slow and cumbersome to open and load numerous subjects, making it impractical to grade large numbers of subjects using this software. An alternate method to enable efficient grading and viewing of images has been developed. First, the composite image should be opened using the MetaFlash or like software. The "Solo" mode should be chosen and the images rotated and turned to obtain the optimal view for an individual subject. The Print Screen option from Windows should then be used to obtain a "screen grab", which should be placed in the "clipboard". The screen image captured will preferably retain the depth cueing that provides for three-dimensional visualization.

Separately, Adobe PhotoShop may be launched and a new file opened. The paste command may be used to create an image of the clipboard. The cropping function may be used to properly size the face and remove the unwanted screen items. This image should be saved as a jpeg or like file with the prefix of the subject number with a B or A for before or after product usage.

After jpeg files is obtained for all subjects in PhotoShop, a new file should be created in the software application MicroSoft® PowerPoint. The jpeg files should be directly inserted onto a PowerPoint slide.
The inserted photo should be expanded to full screen size using the corner arrows. The before and after images may then be checked for optimum alignment by toggling between slides. Slight adjustments may be made as needed with the move tool to obtain the best overlay of the images. The PowerPoint file should then be saved with the page numbers activated to aid in subject identification.

The ability of this technique to create very precise overlays of "before" and "after" images can be very advantageous when monitoring changes in parameters like tonality that have traditionally been performed with two-dimensional photography, but which cannot adequately correct for all positioning issues.

The software used and the file formats are very different for the PRIMOS device. The files are much larger and provide extremely high resolution. We chose different software to process these files.

The processed baseline and subsequent images may be imported into the Rapidform 2000^{™} Plus Pack 3 software (from INUS Technology, Inc.) for further analysis. The two images may then be overlaid on top of each other utilizing various registration and alignment functions.

A pair of points may then be chosen that match on the two images (e.g., the tip of the nose on the before and after images). After choosing three or more pairs of matching points, the analyst should right-click on the images and choose "Done". The software will then nicely overlay the two images on top of each other.

In addition to computer monitors, toggling between before and after images can be accomplished with other devices. Digital picture frames have become available which enable viewing of digital images without the need for a separate computer. These devices contain the necessary components to accept digital images from memory cards, telephone lines or standard cables. After loading the images, the user can choose appropriate viewing parameters on the device and view the changes between before and after images. Handheld devices known as personal data assistants ("PDAs") can also be used to view digital images in a similar manner. Portable DVD players have become available which can display images on a monitor.

Another method of visualization includes the use of lenticular printing, which also enables visualization of three dimensional changes. This method simplifies the sharing of information and can be useful for advertising purposes. Lenticular is a special process for printing that can show depth, motion or both. Lenticular material has ridges for the lenses. Multiple images are interlaced together. The interlaced image is printed behind the lenticular material. The lenses are designed to hide all but one image at a particular angle. As the lenticular print is rotated, the images seen by your eye change. In this way, a series of images seen with lenticular technology creates an animation effect. Lenticular prints can be prepared lithographically or photographically for higher resolution. The photographic method was used to create lenticular prints from the before and after images obtained with the Minolta 3D 1500 camera to demonstrate the product benefits.

A model of the three dimensional surface can be created using a fused deposition modeling system, such as Stratasys Inc. FDM 2000, or other similar equipment.
This allows for visualization of three-dimensional changes while adding a tactile component to the experience.

### Generating a Color Deviation Map:

Using Rapidform 2000^{™}, a color deviation map of the subjects' face may be generated (i.e., an image of the face where different colors denote various levels of depth changes from the baseline image). The levels of changes may be evaluated by subtracting the depth of the different areas on the "after" images from the corresponding areas on the "before" image. The .mdl file containing the before and after images overlaid together was opened. The "Shell/Shell Deviation" function was used. "Post-treatment" and "pre-treatment" or "baseline shell" should be chosen and a deviation map generated for the two shells chosen. On a color deviation map, green represents no changes in depth for a particular area. Bright red denotes maximum elevation or lifting of an area, whereas bright blue implies maximum depression of an area relative to the baseline. The other colors (e.g., orange, yellow) represent intermediate levels of elevation or depression. Presence of "white" in a region means that the amount of change in that area exceeds what the color scale can represent. In this case, the magnitude of the "Minimum Range" and "Maximum Range" settings may be adjusted as needed for the best color distribution.
By looking at the color deviation map and the values of the settings, it can be observed whether the product had, e.g., lifting and contouring effects on certain areas of the face.

### Estimating Cheek Slimming Using a Volume Estimation Software Routine:

IDL surface viewing routines may be used to choose the areas of changes around the cheek. The area should be exported as .obj file. The IDL surface viewing routine may then be used to calculate volume changes. The extent of cheek slimming may be determined as the volume difference between the before and after shells.

### Example 1 - Clinical Study

Thirty-three subjects were enrolled in a controlled study of a cosmetic product at the clinical test site in N.J. during September 2000. Instrumental measurements were taken at baseline and after product usage. Subjects were given a supply of product to use once a day at home for one week. The subjects returned to the Center one week later. Instrumental measurements were taken after product application. The measurements included: Minolta 3D 1500 Camera and Primos 3D Optical Scanning.

### Documenting Visible Benefits of the Product

Two skin care experts were asked to view a presentation of the images. This presentation was made before and then after product usage with increasing sequential subject numbers. The purpose of this presentation was to specifically identify and document visible benefits observed during a controlled product usage situation. The results are shown in Table 1.

**Table 1**

| Improvements | Mean % Subjects Demonstrating Effect |
|---|---|
| Eye Area | 88 |
| Lifting Of Face | 85 |
| Lifting In Eye Area | 85 |
| Better Facial Contour | 78 |
| Lifting Eyebrow Area | 62 |
| Alert Looking | 67 |
| Eyelid Fold / Lines Raised | 67 |
| Slimmer Look | 64 |
| More Defined Contour | 58 |
| Forehead Lines Up / Lift | 61 |
| More Defined Cupid's Bow | 60 |
| Eye Open | 48 |

### Quantifying Slimming of Facial Contour in This Study

The expert graders clearly noted evidence of facial slimming in this study. One of our goals was to quantify this type of effect. We investigated the possibility of using image processing software to detect facial contour edges from the MetaFlash® photographic images and to make quantitative measurements. Adobe® PhotoShop® , a widely used photographic software package was utilized. One of the capabilities of the software is to trace the edges or contours of an object. Settings for the TRACE CONTOUR tool were optimized for our baseline and after product usage images. The resulting images show only the traces of the facial contours detected by the software. This technique eliminates inaccuracy and subjective biases by having the software detect the contours.

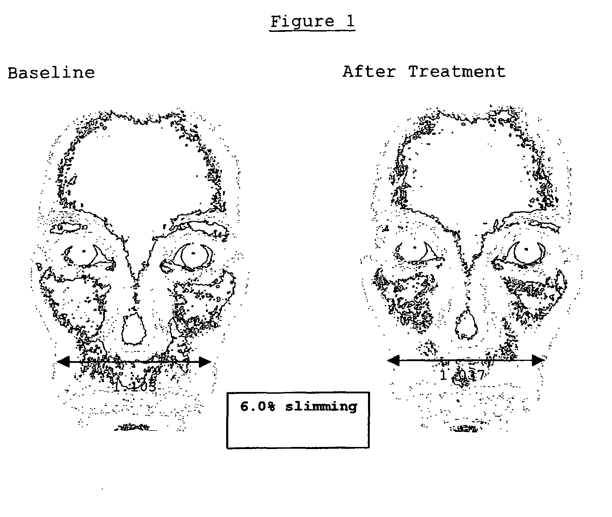

Using the "Measuring tools" 11 of the software, we then evaluated the width of the facial contour. In order to standardize the location of the width measurement across different subjects, we chose to make the measurement at the same level as the inflection points of the two side of the face. The widths of the facial contour before and after treatment were compared and the percentage change was calculated as shown in Figure 1.

### Quantification of Eye Opening Effects in This Study

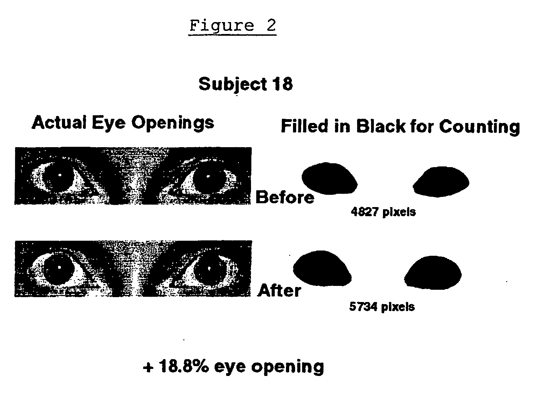

The expert graders also noted evidence of increased eye opening in this study. We investigated the possibility of using image processing software to quantify the change in eye opening observed in this study. Adobe® PhotoShop® , a widely used photographic software package was again utilized. A special PhotoShop® drawing tool called the "magnetic lasso" was used to outline the eye opening area on both eyes in the baseline and after product usage images. This tool is designed to detect the edges of objects, such as the eye opening, and sets anchor points as it is being traced by the analyst. In this way, the software helps to choose the area being measured, thereby eliminating inaccuracy or bias from the measurement. Settings for the "magnetic lasso" tool were optimized for the detection of the eye area in our images. The individual eye areas were then completely filled with a black color to enable quantification of the area. The pixel count for this area was compared in baseline and after product usage images and a percentage increase was calculated as shown in Figure 2.

### Primos 3D Non-contact Optical Scanning for Visualization and Quantification of Lifting and Contouring

The baseline and after product usage images were then imported into Rapidform 2000^{™} Plus Pack 3 software (from INUS Technology, Inc.). This software enabled us to overlay and align the before and after shells with a process called registration. The high-resolution shaded surfaces can then be viewed as three-dimensional renderings.

### Creating Color Deviation Maps

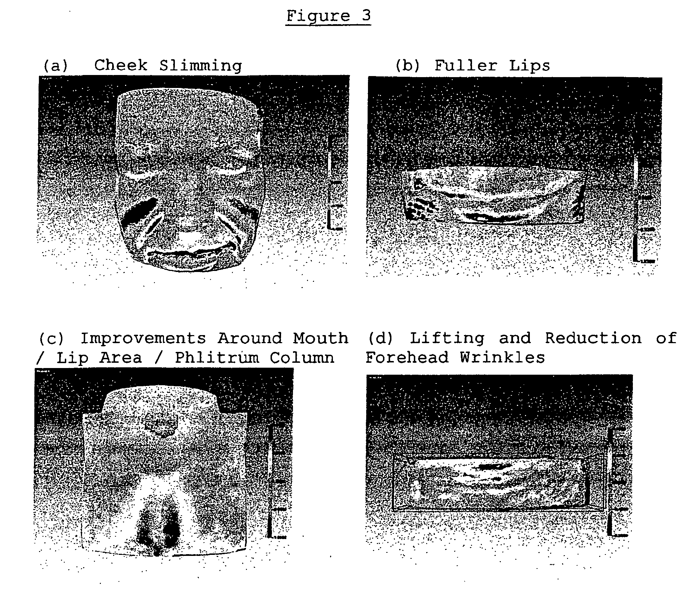

The registered shells were analyzed using Rapidform 2000^{™} to generate a color deviation map of the subject's face as described above. By looking at the color deviation map and the values of the settings, we observed the areas of the face where the product had provided lifting and contouring effects. The investigators viewed these color deviation maps and observed lifting and contouring effects in the expected areas of the face. To document these facial topographical changes after treatment and to validate this new methodology, a subset of subjects that showed evident signs of specific product effects were selected and their color deviation maps were generated. Individual examples are shown below in Figure 3:

This color deviation mapping routine was specifically developed to document cosmetic product effects. These images clearly indicate topographical changes in the areas of the cheek, lips and forehead after product usage. The calculated values for cheek slimming in mm were 1.4 and 2.0 mm, while lip plumping were 1.5 and 0.9 mm, philtrum column changes were 0.8 and 1.6 mm, and forehead wrinkle reduction were 0.9 and 1.0mm.

### Estimating the Volume of Cheek Slimming/Contouring

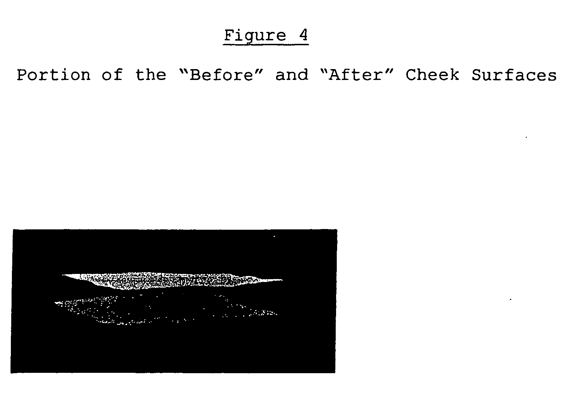

IDL® software was used to determine a quantitative volume for cheek slimming. Subjects that demonstrated clear signs of cheek slimming had the blue areas in their color deviation map. A small rectangular section of this blue area was chosen for analysis. Volumes changes per mm² were calculated to be 1.1 and 1.3 mm³ for a subset of two subjects from this study as shown in Figure 4.

## Claims

1. A method for measuring visible changes in a portion of a human body including:
obtaining a first three-dimensional image of the portion of a human body;
obtaining a second three-dimensional image of the portion of a human body after changes therein;
overlaying the first three-dimensional image and the second three-dimensional image; and
comparing the first and second images to measure visible changes in the portion of a human body.

2. The method according to claim 1 wherein the measurement is obtained from a visualization.

3. The method according to claim 2 wherein the visualization is accomplished utilizing lenticular printing.

4. The method according to claim 2 wherein the visualization is accomplished utilizing a digital picture frame.

5. The method according to claim 2 wherein the visualization is accomplished utilizing a personal data assistant.

6. The method according to claim 2 wherein the visualization is accomplished utilizing a portable DVD player.

## Patentansprüche

1. Verfahren zum Messen sichtbarer Veränderungen in einem Abschnitt eines menschlichen Körpers umfassend:
Erhalten eines ersten dreidimensionalen Bildes des Abschnitts des menschlichen Körpers;
Erhalten eines zweiten dreidimensionalen Bildes des Anschnitts des menschlichen Körpers nach Veränderungen darin;
Übereinanderlagern des ersten dreidimensionalen Bildes und des zweiten dreidimensionalen Bildes; und
Vergleichen des ersten und zweiten Bildes, um sichtbare Veränderungen in dem Abschnitt des menschlichen Körpers zu müssen.

2. Verfahren nach Anspruch 1, **dadurch gekennzeichnet, daß**
die Messung durch eine Visualisierung erhalten wird.

3. Verfahren nach Anspruch 2, **dadurch gekennzeichnet, daß**
die Visualisierung durch Verwenden eines Linsenrasterdrucks erreicht wird.

4. Verfahren nach Anspruch 2, **dadurch gekennzeichnet, daß**
die Visualisierung durch einen digitalen Bilderrahmen erreicht wird.

5. Verfahren nach Anspruch, 2 **dadurch gekennzeichnet, daß**
die Visualisierung durch Verwenden eines Minicomputers erreicht wird.

6. Verfahren nach Anspruch 2, **dadurch gekennzeichnet, daß**
die Visualisierung durch Verwenden eines tragbaren DVD-Spielers erreicht wird.

## Revendications

1. Méthode de mesure des changements visibles dans une partie d'un corps humain comprenant :
l'obtention d'une première image tridimensionnelle de la partie d'un corps humain ;
l'obtention d'une seconde image tridimensionnelle de la partie d'un corps humain après changements dans celle-ci ;
la superposition de la première image tridimensionnelle et de la seconde image tridimensionnelle ; et
la comparaison des première et seconde images pour mesurer les changements visibles dans la partie d'un corps humain.

2. Méthode selon la revendication 1 dans laquelle la mesure est obtenue à partir d'une visualisation.

3. Méthode selon la revendication 2 dans laquelle la visualisation est réalisée en utilisant l'impression lenticulaire.

4. Méthode selon la revendication 2 dans laquelle la visualisation est réalisée en utilisant un cadre photo numérique.

5. Méthode selon la revendication 2 dans laquelle la visualisation est réalisée à l'aide d'un assistant numérique personnel.

6. Méthode selon la revendication 2 dans laquelle la visualisation est réalisée à l'aide d'un lecteur DVD portable.
